(19) Europäisches Patentamt
European Patent Office
Office européen des brevets

(11) Veröffentlichungsnummer: **0 006 590**
**B1**

# (12) EUROPÄISCHE PATENTSCHRIFT

(45) Veröffentlichungstag der Patentschrift: **14.10.81**

(51) Int. Cl.³: **C 07 C 85/06,** C 07 C 87/62

(21) Anmeldenummer: **79102070.4**

(22) Anmeldetag: **22.06.79**

(54) **Verfahren zur Herstellung von N-Alkylarylaminen.**

(30) Priorität: **30.06.78 CH 7157/78**

(43) Veröffentlichungstag der Anmeldung:
**09.01.80 Patentblatt 80/1**

(45) Bekanntmachung des Hinweises auf die Patenterteilung:
**14.10.81 Patentblatt 81/41**

(84) Benannte Vertragsstaaten:
**AT BE DE FR GB IT NL**

(56) Entgegenhaltungen:
**DE-A-2 324 855**
**DE-A-2 646 379**
**DE-B-2 335 906**
**US-A-3 819 709**
**US-E-29 339**
**INDUSTRIAL AND ENGINEERING CHEMISTRY, Product research and development, Band 16, Nr. 3, 1977**
**A. BALKER et al. »Catalytic Amination of Long Chain Aliphatic Alcohols«**
**Seiten 261 bis 266**

(73) Patentinhaber: **LONZA AG, Gampel/Wallis (CH)**

(72) Erfinder: **Bergamin, Renzo, Dr., Alte Gasse, Ried bei Brig (Kanton Wallis) (CH)**

(74) Vertreter: **Weinhold, Peter, Dr. et al, Siegfriedstrasse 8, D-8000 München 40 (DE)**

EP 0 006 590 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von N-Alkylarylaminen

Es ist bekannt, N-Alkylarylamine durch Umsetzung von Arylaminen mit Alkanolen herzustellen. Als Katalysatoren werden beispielsweise Aluminiumoxid oder Silikate verwendet (DE-PS 693 417, DE-PS 638 756). Diese Katalysatoren haben den Nachteil, daß ihre Aktivität rasch abnimmt und somit die Lebensdauer sehr begrenzt ist. Es sind auch schon als Katalysatoren Trägerstoffe eingesetzt worden, die einen Gehalt an Sauerstoffsäuren des Phosphors oder deren Salze besitzen (DP 617 990). Auch solche Katalysatoren haben nur eine kurze Lebensdauer. Aus der DAS 2 335 906 sind Katalysatoren bekannt, die aus Kieselsäure bestehen, die eine innere Oberfläche von 50 bis 500 m²/g und die einen Gehalt von 0,1 bis 20% an Phosphorsäure haben. Dabei wird dem Katalysator während der Umsetzung kontinuierlich Phosphorsäure oder Phosphorsäurealkylester zugeführt. Der Nachteil dieses Verfahrens besteht darin, daß durch die Anwesenheit von Phosphorsäure, auch wenn es nur geringe Mengen sind, große Korrosionsprobleme im gesamten Reaktorsystem entstehen. Außerdem läßt die Selektivität des Katalysators im Hinblick auf die Herstellung von nur N-Monoalkylarylaminen zu wünschen übrig.

Des weiteren ist aus der deutschen Offenlegungsschrift 2 646 379 bekannt, bestimmte Hydrier- bzw. Dehydrierkatalysatoren, insbesondere solche, die in erster Linie Nickel, Kobalt und Kupfer, Kobalt, Mangan und Phosphorsäure oder Nickel, Kupfer und Chrom auf einem Träger, welcher insbesondere aus Aluminiumoxyd besteht, enthalten, bei der Umsetzung von Alkoholen und Anilin zur Herstellung von N-Alkylanilinen zu verwenden. Dieses Verfahren erfordert jedoch die Anwendung von Drucken über 100 bar. Ferner sind die Umsetzungswerte bei diesen Verfahren nicht zufriedenstellend.

In ähnlicher Weise ist auch bei dem Verfahren der US-PS 3 819 709, bei dem zur Herstellung von N-Methylanilin durch Umsetzung von Anilin und Methanol in flüssiger Phase ein metallisches Kupfer enthaltender Katalysator zum Einsatz gelangt, die Anwendung von Drucken zwischen 30 und 300 at notwendig. Dieses Verfahren wird zweckmäßig in einem Druckautoklaven durchgeführt und stellt eine typische diskontinuierliche Flüssigkeitsphasen-Reaktion dar.

Die Aufgabe der vorliegenden Erfindung war es, ein technisch einfaches Verfahren zur Herstellung von N-Alkylarylaminen, insbesondere zur Herstellung von N-Monoalkylarylaminen, ausgehend von Arylaminen und den entsprechenden Alkanolen, zu finden.

Erfindungsgemäß wird die Lösung der Aufgabe dadurch erreicht, daß man einen Katalysator verwendet, der aus Kupfer besteht, das auf einen silikatischen Träger aufgebracht ist. Geeignete silikatische Träger sind z. B. Kaolin, Wasserglas, Kieselgur, Quarz, Aerosil, Siliziumdioxid, Silicagel, Fuellererden, Mullit, Sillimanit oder Zeolith. Der Katalysator kann aus metallischem Kupfer, aufgebracht auf den silikatischen Träger, oder aus Kupferoxid, das auf den Träger aufgebracht ist, bestehen. Vorzugsweise besitzt der Katalysator eine spezifische Oberfläche von 10 bis 100 m²/g, zweckmäßig 12 bis 25 m²/g.

Vorteilhaft wird der Katalysator im Festbett angewendet. Dabei kommt er geformt, z. B. in Würfel- oder Kugelform, zur Anwendung.

Je nachdem, ob in der Hauptsache N-Monoalkylarylamine oder N-Dialkylarylamine erhalten werden sollen, kann das Molverhältnis Arylamin/Alkanol von 0,5 zu 1 bis 1 zu 20 variieren. Das Verfahren der Erfindung ist vorteilhaft zur Herstellung von N-Monoarylaminen geeignet. In diesem Fall wird pro Mol Arylamin 0,5 bis 2, vorzugsweise 1 bis 1,2 Mol Alkanol angewendet.

Bevorzugt verwendet man aromatische Amine der Formel

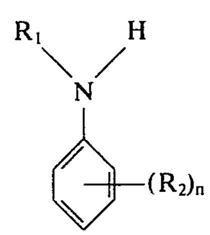

in der $R_1$ für ein Wasserstoffatom oder einen Alkylrest mit 1 bis 4 Kohlenstoffatomen steht, $R_2$ ein Wasserstoffatom, eine Cyano-, oder Nitrogruppe oder einen Alkyl- oder Alkoxyrest mit bis zu 4 Kohlenstoffatomen bezeichnet und n für 1 oder 2 steht und, sofern n = 1 ist, $R_2$ auch für einen Phenylrest oder den Rest der Formel

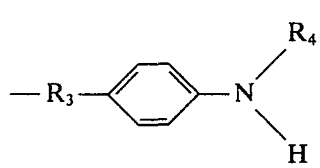

stehen kann, in der $R_3$ für einen Alkylen- oder Alkylidenrest mit bis zu 3 Kohlenstoffatomen steht und $R_4$ ein Wasserstoffatom oder einen Alkylrest mit 1 bis 4 Kohlenstoffatomen bedeuten kann.

Bevorzugt geht man von aromatischen Aminen aus, die sich vom Benzol ableiten und eine Aminogruppe haben. Neben der Aminogruppe können die bevorzugten aromatischen Amine 1 oder 2 unter Reaktionsbedingungen inerte Substituenten, wie Alkylreste mit 1 bis 4 Kohlenstoffatomen, Alkoxyreste mit 1 bis 4 Kohlenstoffatomen, Cyan- oder Nitrogruppen, ferner Halogenatome oder Methylalkoxyreste enthalten. Besonders bevorzugt werden Anilin, Toluidine oder Xylidine als Ausgangsstoffe eingesetzt. Geeignete Amine sind beispielsweise Anilin, o-Toluidin, m-Toluidin, p-Toluidin, Xylidine, Anisidine, kernhalogenierte Aniline oder m- und p-Nitranilin, ferner 4,4'-Diaminodiphenylmethan.

Bevorzugte Alkanole sind solche mit 1 bis 4 Kohlenstoffatomen, wie Methanol, Äthanol, Propanol, Isopropanole oder Butanole. Besondere Bedeutung hat Methanol oder Äthanol erlangt.

Die Umsetzung wird bei einer Temperatur von 180 bis 450°C durchgeführt. Besonders gute Ergebnisse erhält man bei Temperaturen von 220 bis 350°C. In der Regel führt man die Reaktion bei Normaldruck aus. Es ist auch möglich, schwach erhöhten Druck, z. B. bis zu 10 bar anzuwenden. Ferner werden die Temperatur- und Druckbedingungen so gewählt, daß die Umsetzung in der Gasphase durchgeführt werden kann. Zusätzlich hat es sich als vorteilhaft erwiesen, Trägergase, die unter Reaktionsbedingungen inert sind, wie Stickstoff, mitzuverwenden.

Das Verfahren der Erfindung wird vorteilhaft so durchgeführt, daß der Katalysator in einen Festbettreaktor eingebracht wird, auf Reaktionstemperatur aufgeheizt wird und man die Mischung Arylamin/Alkanol während einer kurzen Verweilzeit, z. B. 4 bis 100 sec., eingeleitet wird. Das gasförmige Reaktionsgemisch wird kondensiert, die organische Schicht von der wäßrigen Schicht abgetrennt und aus der organischen Schicht das gewünschte Alkylarylamin isoliert.

## Beispiel 1

In einem elektrisch beheizten Festbettreaktor (∅ 26 mm, Länge 700 mm) wurden 200 Gewichtsteile Katalysator gegeben. Der Katalysator in Würfel- oder Kugelform besteht aus Kupfer auf einem Silikatträger.

Der Katalysator wurde bei 250°C gehalten und stündlich 29,4 Gewichtsteile einer Mischung Methanol/Anilin (Molverhältnis 1 zu 1) zugepumpt.

Die Verweilzeit im Katalysatorbett betrug 42 sec.

Das erhaltene gasförmige Reaktionsgemisch wurde kondensiert. Man erhielt stündlich 26,7 Gewichtsteile Kondensat, das sich in eine wäßrige und organische Schicht auftrennte. Die organische Schicht enthielt 21,4 Gewichtsteile N-Methylanilin, 0,2 Gewichtsteile N,N-Dimethylanilin, 3 Gewichtsteile Anilin und 0,2 Gewichtsteile Methanol. Dies entspricht einem Anilin-Umsatz von 86%. Die Ausbeute an N-Methylanilin, bezogen auf umgesetztes Anilin, betrug 97,7%.

## Beispiele 2–5

| Arylamin | Alkanol | Molverhältnis | Katalyt g | Temperatur °C | Umsatz % | Monoprod. %*) | Diprod. %*) |
|---|---|---|---|---|---|---|---|
| Anilin | N-Butanol | 1 : 1 | 155 | 230 | 72,8 | 80,4 | – |
| m-Toluidin | Äthanol | 1 : 1,2 | 200 | 280 | 80,6 | 85,4 | – |
| Anilin | n-Propanol | 1 : 1 | 155 | 250 | 71,8 | 81,7 | 0,6 |
| Xylidin | Methanol | 1 : 1,5 | 125 | 230 | 58,6 | 97,4 | – |

*) bezogen auf den Umsatz.

**Patentansprüche**

1. Verfahren zur Herstellung von N-Alkylarylaminen durch Umsetzen von Arylaminen mit Alkanolen in der Gasphase bei Temperaturen von 180 bis 450°C in Gegenwart von Katalysatoren, dadurch gekennzeichnet, daß man Arylamine der Formel

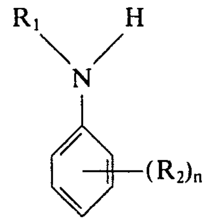

in der $R_1$ für ein Wasserstoffatom oder einen Alkylrest mit 1 bis 4 Kohlenstoffatomen steht, $R_2$ ein Wasserstoffatom, eine Cyano- oder Nitrogruppe, ein Halogenatom oder einen Alkyl-, Alkoxy- oder Methylalkoxyrest mit bis zu 4 Kohlenstoffatomen bezeichnet und n für 1 oder 2 steht und, sofern $n = 1$ ist, $R_2$ auch für einen Phenylrest oder den Rest der Formel

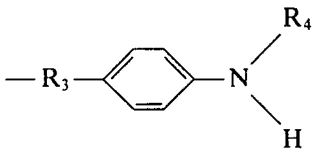

stehen kann, in der $R_3$ für einen Alkylen- oder Alkylidenrest mit bis zu 3 Kohlenstoffatomen steht und $R_4$ ein Wasserstoffatom oder einen Alkylrest mit 1 bis 4 Kohlenstoffatomen bedeuten kann, mit Alkanolen mit 1 bis 4 Kohlenstoffatomen im Molverhältnis von 0,5 zu 1 bis 20 einsetzt und als Katalysator Kupfer, auf Silikatträger aufgebracht, verwendet.

2. Verfahren gemäß Patentanspruch 1, dadurch gekennzeichnet, daß man zur Herstellung von N-Monoalkylarylaminen pro Mol Arylamin 0,5 bis 2 Mol Alkanol verwendet.

3. Verfahren gemäß Patentansprüchen 1 und 2, dadurch gekennzeichnet, daß man einen Katalysator verwendet, der eine spezifische Oberfläche von 10 bis 100 $m^2/g$ aufweist.

4. Verfahren gemäß Patentansprüchen 1 bis 3, dadurch gekennzeichnet, daß man einen Katalysator verwendet, der aus Kupfer oder Kupferoxid, aufgebracht auf einen Kaolin-Wasserglasträger, besteht.

5. Verfahren gemäß Patentansprüchen 1 bis 4, dadurch gekennzeichnet, daß man die Umsetzung bei einem Druck zwischen Normaldruck und einem Druck bis zu 10 bar durchführt.

**Claims**

1. Process for the production of N-alkylaryl amines by reacting aryl amines with alkanoles in the gaseous phase at temperatures from between 180 to 450° C in the presence of catalysts, characterized in starting from arylamine having the formula

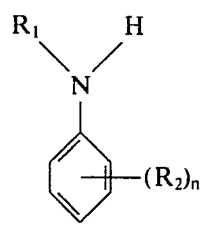

wherein $R_1$ is a hydrogen atom or an alkyl radical having from 1–4 carbon atoms, $R_2$ is a hydrogen atom, a cyano or nitro group, a halogen atom or an alkyl, alkoxy or methyl alkoxy radical having up to 4 carbon atoms, and n is 1 or 2, and under the proviso that $n = 1$, $R_2$ may also be a phenyl radical or a group having the formula

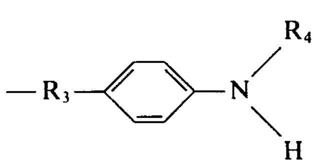

wherein $R_3$ is an alkylene or alkylidene radical having up to 3 carbon atoms, $R_4$ is a hydrogen atom or a

alkyl radical having from 1 – 4 carbon atoms, together with alkanols having from 1 – 4 carbon atoms in a molar ratio of from 0.5 to 1 – 20, using as catalyst copper deposited on silicate support.

2. Process according to claim 1, characterized in using for the production of N-monoalkylaryl amines 0.5 to 2 moles alkanole per mole arylamine.

3. Process according to claims 1 and 2, characterized in using a catalyst having a specific surface area of from 10 – 100 m²/g.

4. Process according to anyone of claims 1 to 3, characterized in using a catalyst consisting of copper or copper oxide deposited on a kaolin/waterglass support.

5. Process according to anyone of claims 1 to 4, characterized in that the reaction is carried out at a pressure from between normal pressure to 10 bar.

**Revendications**

1. Procédé de préparation de N-alcoylarylamines par réaction d'arylamines acec des alcanols en phase gazeuse à des températures de 180 à 450° C, en présence de catalyseurs, caractérisé en ce qu'on utilise une arylamine de formule:

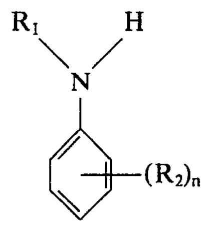

dans laquelle:
$R_1$ représente un atome d'hydrogène ou un radical alkyle ayant de 1 à 4 atomes de carbone;
$R_2$ représente un atome d'hydrogène, un radical cyano ou un groupe nitro, un atome d'halogène, ou un radical alkyle alcoxy ou méthylalcoxy présentant jusqu'à 4 atomes de carbone;
n représente 1 ou 2, et lorsque n représente 1, $R_2$ peut également représenté un radical phényle ou un radical de formule:

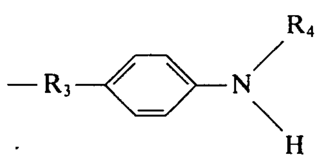

dans laquelle $R_3$ représente un radical alkylène ou alkylidène ayant jusqu'à 3 atomes de carbone; et $R_4$ représente un atome d'hydrogène, ou un radical alkyle ayant de 1 à 4 atomes de carbone, avec un alcanol ayant de 1 à 4 atomes de carbone, dans un rapport molaire de 0,5 – 1 jusqu'à 20, et en tant que catalyseur, on utilise du cuivre sur support de silice.

2. Procédé selon la revendication 1, caractérisé en ce pour la fabrication de N-monoalcoylarylamines, on utilise par mole d'arylamine, 0,5 à 2 moles d'alcanol.

3. Procédé selon l'une quelconque des revendications 1 ou 2, caractérisé en ce que l'on utilise un catalyseur qui présente une tension superficielle spécifique de 10 à100 m²/g.

4. Procédé selon l'une quelconque des revendications 1 à 3, caractérisé en ce que l'on utilise un catalyseur qui comprend du cuivre, ou de l'oxyde de cuivre, porté sur un suport de kaolin – verre liquide.

5. Procédé selon l'une quelconque des revendications 1 à 4, caractérisé en ce que l'on effectue la transformation sous une pression comprise entre la pression atmosphérique ou une pression allant jusqu'à 10 bars.